# EUROPEAN PATENT SPECIFICATION

(11) **EP 1 095 274 B1**
(45) Date of publication and mention of the grant of the patent: **29.09.2010**
(21) Application number: 99935251.1
(22) Date of filing: 07.07.1999
(51) Int. Cl.: G01N 33/543, A61K 39/00, A61K 47/48, A61K 51/00, A61K 49/00

(54) **TRIFUNCTIONAL REAGENT FOR CONJUGATION TO A BIOMOLECULE**
TRIFUNKTIONALES REAGENZ ZUR VERBINDUNG VON BIOMOLEKÜLEN
REACTIF TRIFONCTIONNEL POUR LA CONJUGAISON AVEC UNE BIOMOLECULE

(30) Priority: 07.07.1998 WO PCT/SE98/01345
(43) Date of publication of application: 02.05.2001
(73) Proprietor: Department of Radiation Oncology, University of Washington, Seattle, WA 98103 (US); Mitra Medical Technology AB, 223 70 Lund (SE)
(72) Inventor: WILBUR, D., Scott, Edmonds, WA 98026 (US); SANDBERG, Bengt, E., B., S-245 62 Hjärup (SE)
(74) Representative: Henriksson, Dan Ragnar Mikael
(86) International application number: PCT/SE1999/001241
(87) International publication number: WO 2000/002051

(56) References cited:
- EP-A1- 0 618 192
- EP-A2- 0 310 361
- WO-A1-89/10140
- WO-A1-93/02105
- WO-A1-96/04313
- WO-A1-97/29114
- WO-A2-99/04820
- US-A- 5 134 071
- US-A- 5 310 916
- PROC. NATL. ACAD. SCI. VLADIMIR R. MUZYKANTOV ET AL: 'Immunotargeting of Antioxidant Enzymes to the Pulmonary Endothelium' vol. 93, May 1996, USA, pages 5213 - 5218, XP002921089
- CHEMICAL ABSTRACTS, vol. 126, no. 20, 19 May 1997, Columbus, Ohio, US; abstract no. 126:258425R, OTUSJI EIGO ET AL: 'Decreased Renal Accumulation of Biotinylated Chimeric Monoclonal Antibody-Neocarzinostatin Conjugate After Administration of Avidin' page 17; XP002921090 & J. CANCER RES. vol. 88, no. 2, 1997, ENG, pages 205 - 212
- THE JOURNAL OF IMMUNOLOGY TUTT ALISON ET AL: 'Trispecific F(ab')3 Derivatives that Use Cooperative Signaling Via the TCR/CD3 Complex and CD2 to Activate and Redirect Resting Cytotoxic T Cells' vol. 147, no. 1, 01 July 1991, USA, pages 60 - 69, XP000863699

## Description

### Field of Invention

The present invention is directed to a reagent for the conjugation to a biomolecule, to a conjugate containing said reagent, to a method for the production of said conjugate, to a method for in vitro analysis of affinity labelled biomolecules; and to a kit comprising said reagent.

More precisely, the present invention is generally directed at a novel chemical reagent which simultaneously conjugate an affinity ligand and an effector agent with a biomolecule to obtain minimal modification of that biomolecule.

As an example, chemical reagents which contain an affinity ligand (e.g. a biotin moiety), an effector agent (e.g. a radiolabeling moiety), and a biomolecule reactive moiety are coupled together through a trifunctional cross-linking moiety and spaced apart with linker moieties. Using such a reagent, a biomolecule can be biotinylated and radiolabeled via one of two methods, then employed in medical protocols, such as those utilizing extracoporeal immunoabsorptive removal methods to minimize the toxic effects to normal tissue and blood components.

### Background of the Invention

Many biomolecules, including proteins and peptides, hold potential as reagents for use in diagnosis and therapy of human conditions and diseases. As most biomolecules do not, by themselves, have properties to make them useful as diagnostic and/or therapeutic reagents, bio-molecules of interest are often chemically modified to achieve this. However, one very important criterion must be applied when chemically modifying biomolecules. That criterion is that the modification does not alter the biological property that is important (e.g. cancer cell targeting) in the use of that particular biomolecule. This criterion makes it imperative that site-selective (where possible) and minimal modification of the biomolecule be conducted.

Modification of a targeting biomolecule with an effector agent, such as a radionuclide, can provide valuable new tools for diagnosis and therapy of human and animal diseases or conditions. Coupling of a radionuclide to the biomolecule results in the desired diagnostic effect of providing photons that can be measured or imaged externally to show the localization of the radiolabeled biomolecule, or it may provide the desired therapeutic effect of causing damage to cells or tissues that are targeted by the biomolecule. Biomolecules labeled with photon emitting radionuclides can be used for the diagnosis of a number of human conditions (i.e. extent of myocardial infarcts, presence of cancer, etc.). For example, technetium-99m labeled antibodies can be used to diagnose cancer (Granowska et al. Eur. J. Nucl. Med. 20, 483-489, 1993; Lamki et al. Cancer Res. 50, 904s-908s, 1990; Goldenberg et al. Cancer Res. 50, 909s-921s, 1990); iodine-123 labeled fatty acids can be used to evaluate myocardial perfusion (Corbett J. Nucl. Med. 35, 32s-37s, 1994; Hansen J. Nucl. Med. 35, 38s-42s, 1994; Knapp et al. J. Nucl. Med. 36, 1022-1030, 1995); and fluorine-18 labeled fluorodeoxyglucose can be used to evaluate a variety of functions of the brain (Posner et al., Science 240, 1627-1631, 1988). Biomolecules labeled with particle emissions (e.g. beta, positron, alpha, Auger electrons) can potentially be used for targeted radiotherapy of human disease such as cancer. For example, a large number of monoclonal antibodies (Behr et al. J. Nucl. Med. 38, 858-870, 1997; Divgi et al. J. Nucl. Med. 36, 586-592, 1995; DeNardo et al. Anticancer Res. 17, 1735-1744, 1997) and peptides (Zamora et al. Int. J. Cancer 65, 214-220, 1996; Stolz et al. Digestion 57, 17-21, 1996; Bender et al. Anticancer Res. 17, 1705-1712, 1997) labeled with therapeutic radionuclides such as iodine-131, yttrium-90 and Re-188 are being investigated as new reagents for cancer therapy. Thus, an important modification that can be carried out is to attach a functional moiety to the biomolecule which binds or bonds with a radionuclide. For small (i.e. < 2000 Da molecular weight) biomolecules, usually only one radionuclide binding/bonding moiety is site-selectively attached to cause minimal perturbation in its desired biological properties. Larger biomolecules, such as peptides and proteins, may be conjugated with more than one radionuclide binding/bonding moiety before loss of the desired biological properties, but these molecules generally retain more of their desired biological properties when minimal number of conjugations are obtained.

Modification of biomolecules with an "affinity ligand" is also important as it provides a means of coupling two entities together for a variety of *in vitro* and *in vivo* applications. By their nature, affinity ligands come in pairs. The preferred affinity ligands used for coupling to the biomolecule must have a high enough binding constant (e.g. 10⁶ M⁻¹ or greater) with a second compound to allow the two coupled entities to remain together for a period of time. An example of an affinity ligand pair is a monoclonal antibody and its hapten. The affinity ligand pairs of biotin/avidin and biotin/streptavidin are often used with biomolecules. The very strong interaction (i.e. K = 10¹³ - 10¹⁵ M⁻¹) of biotin with the proteins avidin and streptavidin (Green, Methods Enzymol. 184, 51-67, 1990; Green, Adv. Prot. Chem. 29, 85-133, 1975) provides a foundation for their use in a large number of applications, both for *in vitro* and *in vivo* uses. While the proteins avidin and streptavidin are sometimes conjugated with biomolecules, conjugation of biotin introduces less perturbation of the biomolecule, and more than one biotin molecule can be conjugated with minimal affect on the biomolecule. Therefore, the preferred affinity label is biotin or a derivative thereof, and the examples herein are reflective of this preference. As with the radionuclide binding/bonding moiety, it is important to minimize the number of affinity ligands (e.g. biotin conjugates) attached to a biomolecule to retain the desired biological properties.

Modification of the biomolecule by attachment (conjugation) of another molecule to a particular reactive functional group (e.g. amine, sulfhydryl, aldehyde, ketone) precludes attachment of a second molecule to that group. Thus, if attachment of more than one type of molecule to a biomolecule is desired (to impart two functions), the attachment must be made at a second site using currently available reagents. Since in some applications, it is desirable to have both an affinity ligand and an effector agent (e.g. a moiety that binds/bonds with a radionuclide), site-selective conjugation is precluded. Further, modification of biomolecules that are not made in a site-selective manner (e.g. reaction with surface amine groups in proteins) are limited due to the fact that two different sites are modified. Additionally, modification of larger biomolecules (e.g. proteins) in two subsequent steps can result in a heterogeneous population of modified biomolecules in which molecules that contain the second conjugated species may have less of the desired biological properties (i.e. tumor targeting) than those that do not contain the second conjugate. This can result in a subgroup of biomolecules containing both conjugated species that do not have the properties desired. To circumvent these problems, the affinity ligand (e.g. biotin moiety) and an effector agent (e.g. radionuclide binding/bonding moiety with or without the radionuclide) can be coupled together through trifunctional cross-linking reagent to form a new type of reagent. With the use of this new class of reagents, an equal number of affinity ligands and radionuclide binding/bonding moieties will be conjugated to the biomolecule. With a combined affinity ligand and radiolabeling compound, site specific addition of both reagents can be made, and minimization of the number of conjugates to the biomolecule can be attained. Linking an affinity ligand such as biotin to a fluorescent moiety which is further attached to an oligosaccharide is described in Varki et al., WO 94/28008. The issue of attaching an affinity ligand to cytotoxic agent or an agent which can convert a prodrug to an active drug, and where either of these are further attached to a targeting molecule, is addressed in Nilsson et al., US patent application 08/090 047. However, none of these publications neither alone or in combination describe or indicate the present innovation. The issue of combining an affinity reagent and effector agent on one molecule to achieve minimal modification of biomolecules is not unique to biotin (as the affinity ligand) or radionuclide binding/bonding moieties (the effector agent), and is not limited to only one affinity ligand and one effector ligand per molecule. Combinations of more than one affinity ligand and/or more than one effector per molecule may be advantageous for certain applications.

The radiolabeled and affinity ligand conjugated biomolecule products obtained from this invention are useful in many *in vitro* and *in vivo* applications. A preferred application, where the biomolecule is a tumor binding monoclonal antibody, toxin conjugate, or enzyme conjugate, the affinity ligand is biotin or a derivative thereof, and the radionuclide is a diagnostic or therapeutic radionuclide used in a patient cancer treatment protocol, is to use a biotin binding (e.g. avidin coated) column for extracorporeal immunoabsorptive removal of a radiolabeled antibody conjugate from a patient's blood. Extracorporeal removal of the radiolabeled antibody, toxin conjugate, or enzyme conjugate limits the toxic effects of the radioactivity, toxin, or enzyme to specifically targeted tissues, minimizing the exposure time and interaction with non-target tissues. Importantly, to be effective, medical agents (e.g. biomolecules) must exert their pharmacological action on a particular target tissue or group of target cells. Targeting of such agents is most often carried out by systemic administration (i.e. intravenous injection) which means that they will be transported through the blood and lymph system to most parts of the body. This transportation, or circulation, of the medical agent throughout the body can result in undesirable toxic side effects in tissues or organs other than those where the effect of the agents is beneficial to the patient.

Specific tissue or organ localization of a medical agent is a very important factor in its effective application. Lack of specific tissue localization is of particular importance in the treatment with medical agents where the desired effect is to kill certain types of cells such as in the treatment of cancer. In order to increase the specificity and thereby make the cancer therapy more effective, tumor marker specific targeting agents such as cancer cell binding monoclonal antibodies have been used as carriers for various cell toxic agents (immunoconjugates) such as, but not limited to, radionuclides, cytotoxins, and enzymes used in prodrug protocols (Meyer et al., Bioconjugate Chem. 6, 440-446, 1995; Houba et al., Bioconjugate Chem. 7, 606-611, 1996; Blakey et al., Cancer Res. 56, 3287-3292, 1996). Although, monoclonal antibodies are selectively bound with tumor cells over non-tumor cells, an initial high concentration of the toxic immunoconjugate is required to optimize binding of a particular agent with tumors in a patient. While required for optimal therapy of the cancer, the high concentration of cytotoxic material in blood and non-target tissues causes undesirable side-effects on sensitive and vital tissues like the bone marrow. Various methods have been proposed to rapidly clear these agents from blood circulation after that the tumor has received a maximum dose of the immunoconjugate. Some blood clearance methods involve the enhancement of the bodies own clearing mechanism through the formation of various types of immune complexes. Similarly, blood clearance can be obtained by using molecules that bind with the immunoconjugate, such as monoclonal antibodies (Klibanov et al., J. Nucl. Med. 29, 1951-1956, 1988; Marshall et al., Br. J. Cancer 69, 502-507, 1994; Sharkey et al. Bioconjugate Chem. 8, 595-604, 1997), (strept)avidin (Sinitsyn et al., J. Nucl. Med. 30, 66-69, 1989; Marshall et al., Br. J. Cancer 71, 18-24, 1995), or biotin containing compounds which also contain sugar moieties recognized by the asialoglycoprotein receptor on liver cells (Ashwell and Morell, Adv. Enzymol. 41, 99-128, 1974). Other methods involve means of removing the circulating immunoconjugates through extracorporeal methods (see review article by Schriber G.J. & Kerr DE, Current Medicinal Chemistry, 1995, Vol. 2, pp 616-629).

The extracorporeal techniques used to clear a medical agent from blood circulation is particularly attractive. Extracorporeal devices for this application have been described (Henry CA, 1991, Vol.18, pp.565; Hofheinz D et al, Proc. Am. Assoc. Cancer Res. 1987 Vol. 28, pp. 391; Lear JL, et al. Radiology 1991, Vol.179, pp.509-512; Johnson TK, et al. Antibody Immunoconj. Radiopharm. 1991, Vol. 4, pp.509; Dienhart DG, et al. Antibody Immunoconj. Radiopharm. 1991, Vol. 7, pp.225 ; DeNardo GL, et al. J. Nucl. Med. 1993, Vol. 34, pp. 1020-1027 ; DeNardo GL, et al. J. Nucl. Med. 1992b, Vol. 33, pp. 863-864; DeNardo S.J., et. al. J. Nucl. Med. 1992a, Vol. 33, pp. 862-863. U.S. Patent Number 5,474,772; Australian patent 638061, EPO 90 914303.4 of Maddock, describe these methods.

To make the blood clearance more efficient and to enable processing of whole blood, rather than blood plasma, the medical agent (e.g. tumor specific monoclonal antibody carrying cell killing agents or radionuclides for tumor localization) have been biotinylated and cleared with the use of an affinity (e.g. biotin-binding) column. A number of publications provide data which show that this technique is both efficient and practical for the clearance of biotinylated and radionuclide labeled tumor specific antibodies (Norrgren K, et al. Antibody Immunoconj Radiopharm 1991, Vol. 4, pp. 54; Norrgren K, et. al. J. Nucl. Med. 1993, Vol. 34, pp. 448-454 ; Garkavij M, et. al. Acta Oncologica 1996, Vol. 53, pp.309-312; Garkavij M, et. al. J. Nucl. Med. 1997, Vol. 38, pp. 895-901). U.S. Patent application No. 08/090,047, EPO 92 903 020.3 of Nilsson and 08/434,889 of Maddock describe these applications.

### Summary of the Invention

The object of the present invention is to eliminate the above mentioned problems in the art. This object is achieved with a reagent as described by way of introduction and having the features defined by the characterising part of claim 1. Preferred embodiments are presented in the subclaims.

In one embodiment the present invention refers to use of said reagent for the manufacture of a medicament for diagnosis and/or treatment of cancer.

In another embodiment the present invention refers to a conjugate comprising said reagent.

In a further embodiment the present invention relates to a method for the production of said conjugate.

In still another embodiment the present invention refers to a method for the production of a radiolabelled conjugate having the features defined in claim 23.

In still another embodiment the present invention refers to a method for in vitro analysis of affinity labelled biomolecules having the features defined in claim 24.

In still another embodiment the present invention refers to a kit for extracorporeally eliminating or at least reducing the concentration of a non-tissue-bound therapeutic or diagnostic biomolecule conjugate in the plasma or whole blood of a vertebrate host, said kit further having the features defined in claim 25.

In general, the invention discloses a new type of compound which combines an affinity ligand and an effector agent in a single molecule that can be used to modify biomolecules. The modified biomolecules are themselves new entities in that fewer sites on them are modified than obtainable with previous reagents. More specifically, the invention describes the chemical components and examples of a new type of molecule (shown in schematic structure (I)) that can be used to conjugate an affinity ligand, such as biotin, and concurrently conjugate an effector ligand, such as a radionuclide binding/bonding moiety with/without a radiolabel, to a biomolecule of interest for a variety of diagnostic and therapeutic applications. This invention also discloses two approaches to the attaching both affinity ligands and radionuclides to a biomolecule (i.e. preformed and postformed labeling approaches) in accordance to the routes shown in Scheme II. For therapeutic applications, a pre-ferred method of blood clearance of the new medical agent (conjugated biomolecule), using extracoporeal immunoabsorptive columns is disclosed.

Further, the new reagent according to the present invention can also be used for in vitro analysis of affinity labelled biomolecules, e.g. monoclonal antibodies or derivatives thereof, labelled with e.g. biotin or derivatives thereof. Thus, due to the presence of a photoactive agent, e.g. a chromophore or a fluorophore, as effector agent in the reagent molecule, it is possible to determine the amount of affinity label bound to the biomolecule as this amount is proportioned to the amount of photoactive agent.

### Detailed Description

*General structure of compounds disclosed.* The chemical nature of a compound for concurrent conjugations of an affinity ligand and an effector agent is shown graphically in the schematic structure (I). A brief description of the various parts of the generalized formulation is provided in the text following the schematic structure (I):

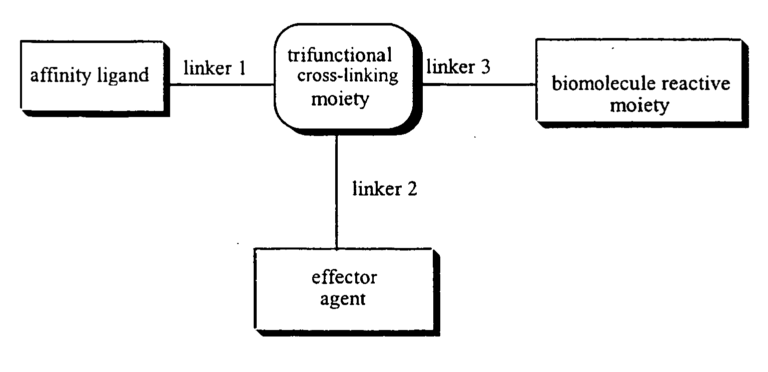

The term "affinity ligand" used throughout the description and the claims means any moiety that binds with another molecule with an affinity constant of 10⁶ M⁻¹ or higher. A preferred affinity ligand is a biotin moiety which can be biotin, or any derivative or conjugate of biotin that binds with avidin, streptavidin, or any other biotin binding species.

The term "effector agent" used throughout the description and the claims means a radionuclide binding moiety with or without the radionuclide, a synthetic or naturally occurring toxin, an enzyme capable of converting pro-drugs to active drugs, immunosuppressive or immunostimulating agents, or any other molecule known or found to have a desired effect, directly or indirectly, on cells or tissues.

The term "biomolecule reactive moiety" used throughout the description and the claims means any moiety that will react with a functional group naturally occurring or synthetically introduced on a biomolecule.

The term "trifunctional cross-linking moiety" used throughout the description and the claims means any chemical moiety that can combine the affinity ligand (e.g. biotin moiety), effector agent (e.g. radionuclide binding/bonding moiety) and a biomolecule reactive moiety.

The term "linker 1" used throughout the description and the claims means a chemical moiety that is an attaching moiety and spacer between the trifunctional cross-linking moiety and the biotin moiety such that binding with avidin or streptavidin, or any other biotin binding species, is not diminished by steric hindrance. Linker 1 may also impart increased water solubility and biotinidase stabilization.

The term "linker 2" used throughout the description and the claims means a chemical moiety that is used to attach the radionuclide binding moiety to the trifunctional cross-linking moiety. Linker 2 may also impart increased water solubility.

The term "linker 3" used throughout the description and the claims means a chemical moiety used to attach the biomolecule reactive moiety to the trifunctional cross-linking moiety. Linker 3 may not be required, but may be advantageous in some cases. Linker 3 may be used as a spacer and/or it may be used to increase the water solubility of the compound.

*Affinity ligand.* The preferred affinity ligand is biotin or a derivative thereof. In most examples the biotin moiety will be natural biotin 1, which is coupled to linker 1 through an amide bond. In some examples it may be advantageous to have a biotin derivative that does not bind as tightly as natural biotin, or a biotin derivative that binds to chemically modified, or genetically mutated, avidin or streptavidin in preference to natural biotin. Examples of such biotins are norbiotin 2, homobiotin 3, oxybiotin 4, iminobiotin 5, desthiobiotin 6, diaminobiotin 7, biotin sulfoxide 8, and biotin sulfone 9. Other modifications of biotin, including further modification of 2 - 9, are also included.

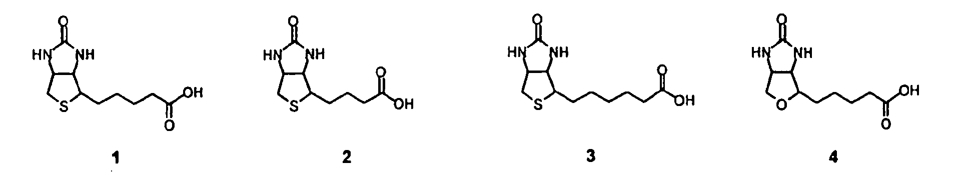

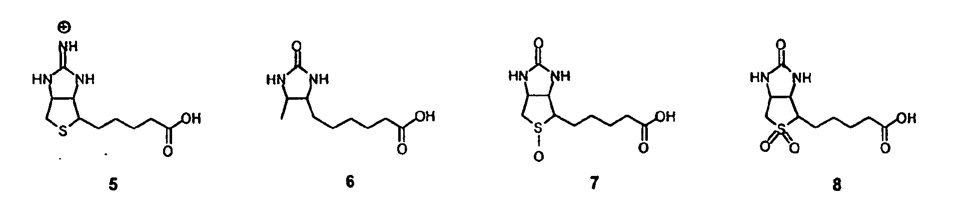

*Effector agent*. The preferred effector agent is a radionuclide binding/bonding moiety, with or without the radionuclide being present. There are a large number of radionuclides that are potentially useful for diagnostic and therapeutic purposes (see articles in Spencer et al. eds., Radionuclides in Therapy, CRC Press, 1987; Ruth et al., Nucl. Med. Biol. 16, 323-336, 1989), and thus moieties which bind or bond with them may be incorporated as the radionuclide binding/bonding moiety. Examples of gamma imaging radionuclides include, Tc-99m, In-111, and I-123. Examples of positron imaging radionuclides include Ga-68, F-18, Br-75, Br-76, and I-124. Examples of therapeutic radionuclides include Y-90, I-131, Re-186, Re-188, Cu-67, Sm-153, Lu-177, Bi-212, Bi-213 and At-211. It is a requirement that the radionuclides be bound by chelation (for metals) or covalent bonds in such a manner that they do not become separated from the biotinylation/- radiolabeling compound under the conditions that the biomolecule conjugates are used (e.g. in patients). Thus, the most stable chelates or covalent bonding arrangements are preferred. Examples of such binding/bonding moieties are: aryl halides and vinyl halides for radionuclides of halogens; N₂S₂ 9 and N₃S 10 chelates for Tc and Re radionuclides; amino-carboxy derivatives such as EDTA 11, DTPA 12, derivatives Me-DTPA 13 and cyclohexyl-DTPA 14, and cyclic amines such as NOTA 15, DOTA 16, TETA 17, CITC-DTPA (not shown, US patent 4,622,420 ), and triethylenetetraaminehexaacetic acid derivatives (not shown, see Yuangfang and Chuanchu, Pure & Appl. Chem. 63, 427-463, 1991) for In, Y, Pb, Bi, Cu, Sm, Lu radionuclides. Attachment of the radionuclide binding/bonding moiety to linker 2 can be achieved at a number of locations in the moieties.

The effector agent can also be a photoactive compound or a compound which can be converted to a photoactive compound, such as a chromophore, fluorophore or any other conventionally used photoactive compound.

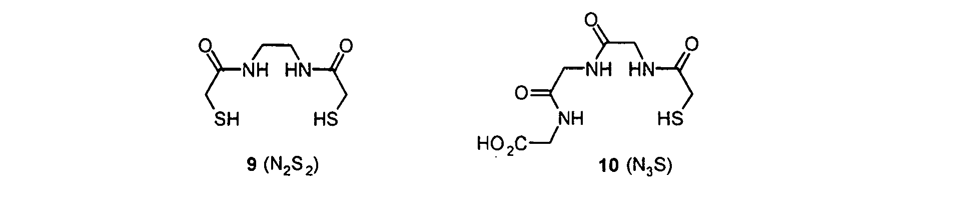

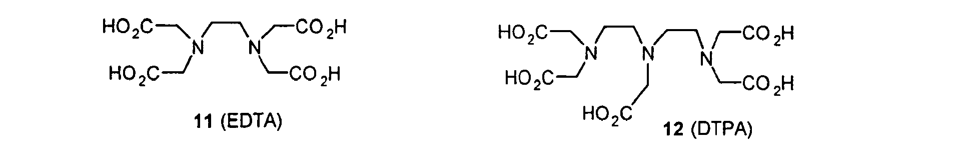

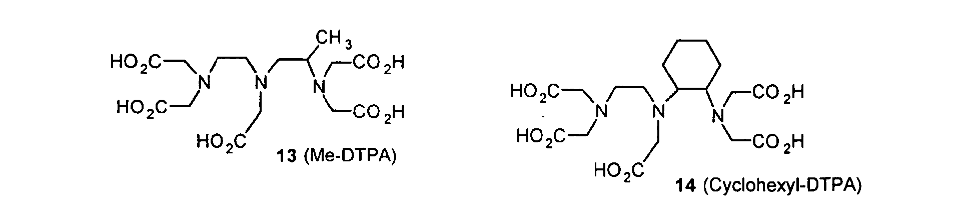

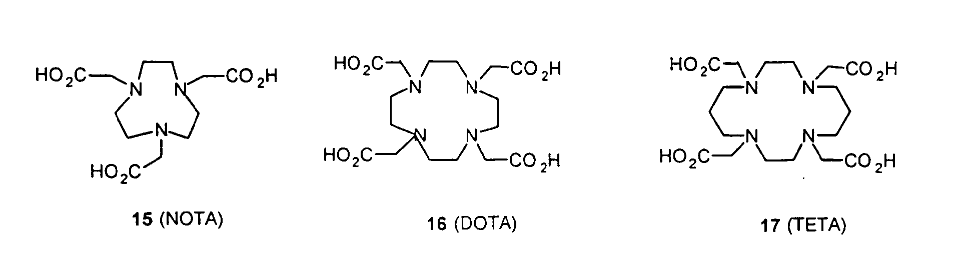

*Biomolecule reactive moiety.* There are a number of moieties that are reactive with functional groups that may be present on a biomolecule, e.g. a protein. For example, aryl or alkyl activated carboxylic acids can be reacted with nucleophilic groups such as primary or secondary amines. Such activated esters include: N-hydroxysuccinimide esters 18, sulfo-N-hydroxysuccinimide esters 19, phenolic esters (e.g. phenol 20, p-nitrophenol 21, tetrafluorophenol 22). Other amine reactive groups include aryl and alkyl imidates 23 and alkyl or aryl isocyanates or isothiocyanates, 24. Sulfhydryl groups on the biomolecule can be reacted with maleimides 25 or alpha-haloamide 26 functional groups. Biomolecules containing naturally occurring or synthetically produced (e.g. by conjugation or from oxidized sugar moieties) aldehydes and ketones can be reacted with aryl or alkyl hydrazines 27, aryl or alkyl acylhydrazines 28, alkyl or aryl hydroxylamines 29.

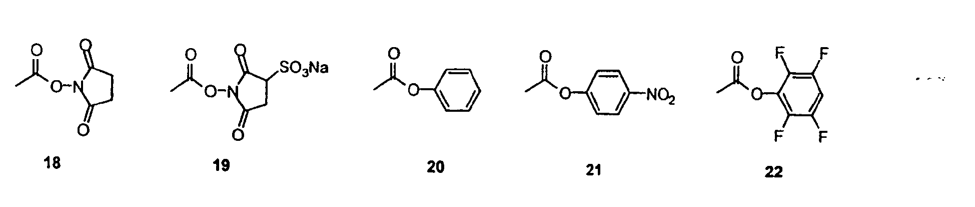

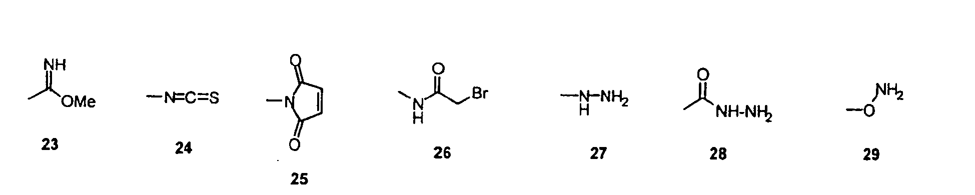

*Trifunctional cross-linking moiety.* The trifunctional cross-linking moiety has two functional groups that can be used to couple with linker 1 and linker 2. It has another functional group that can be either converted directly into the biomolecule reactive moiety or coupled with linker 3. Examples of preferred trifunctional cross-linking moieties are triaminobenzene 30, tricarboxybenzene 31, dicarboxyaniline 32, and diaminobenzoic acid 33. If the functional groups present on the cross-linking 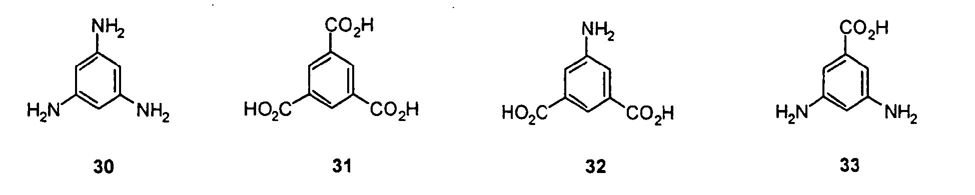 moiety are not by themselves reactive with a functional group on the biomolecule, then they are converted into more reactive moieties, such as activated esters (for carboxylic acids), imidates (cyano functional groups), maleimides (amino), isocyanates, isothiocyanates, etc. The functional groups present on the cross-linking moiety may vary, and protection/deprotection/activation steps may be required to synthesize the desired compound. A trifunctional cross-linking moiety is preferred, but in those examples where more than one effector agent, affinity ligand, or protein reactive moiety is advantageous, tetrafunctional, or higher, cross-linking moieties may be applied.

*Linker moieties.* The linker moieties function as spacers and also may aid in water solubilization for compounds that do not contain ionized or ionizable functionalities. Linker 1 must provide ample space between the biotin moiety and the trifunctional cross-linking moiety such that there is a minimum of 9Å for biotin binding with avidin or streptavidin. Extended linkers (e.g. 6 - 20 atoms in length) are preferred to assure that there is no steric hindrance to binding avidin or streptavidin from the biomolecule that the conjugate is attached to. The extended linkers may contain hydrogen bonding atoms such as ethers or thioethers, or ionizable groups such as carboxylates, sulfonates, or ammonium groups, to aid in water solubilization of the biotin moiety. Many of the biotin moieties are highly insoluble in water. When the compounds of this invention are used in serum or in animals or people, there is an additional requirement for a linker attached to biotin that is not required for linkers attached to other moieties. This requirement is to provide a means of blocking the enzyme biotinidase (Wolf et al., Methods Enzymol. 184, 103-111, 1990; Pipsa, Ann. Med. Exp. Biol. Fenn 43, Suppl. 5, 4-39, 1965) from cleaving the amide bond (biotinamide) to release biotin. This requirement is met by altering the distance between the bicyclic rings of the biotin moiety (as in norbiotin or homobiotin) or using a biotin derivative that has a dramatically decreasing binding with avidin or streptavidin (e.g. desthiobiotin). If natural biotin is used, blockade of biotinidase activity is provided by introducing an alpha carboxylate (Rosebrough, J. Pharmacol. Exp. Ther. 265, 408-415, 1993) or an N-methyl group (Wilbur et al., Bioconjugate Chem. 8, 572-584, 1997) in Linker 1.

Linker 2 must provide a means of coupling an effector agent, such as a radionuclide binding/bonding moiety, with the trifunctional cross-linking moiety. The nature of linker 2 can be highly dependent on the chemistry associated with effector agent employed, partcularily in the case where the effector agent is a radionuclide binding/bonding moiety. Although linker 2 may be as short as 1 atom, it is preferred to have more space than 1 atom provided to decrease the steric environment around the affinity ligand (e.g. biotin moiety). Linker 2 can also have the water solubilizing atoms or groups of atoms to increase water solubility. Linker 3, if required, provides additional space between the biomolecule and the biotin moiety, and can be used to provide additional water solubilization where required. Examples of preferred non-ionized linkers include the trioxadiamine 34 and dioxadiamine 35. Examples of preferred ionized linkers include aryl diaminosulfonate 36 and aryl diamino-trimethylammonium 37. Examples of linkers that also contain a biotinidase blocking moiety are made by combining one of the linkers 34 - 37 with another molecule, for example combining linker 34 with N-methylglycine to yield linker 38, where the N-methyl end must be attached to the biotin moiety to impart stability towards biotinidase cleavage.

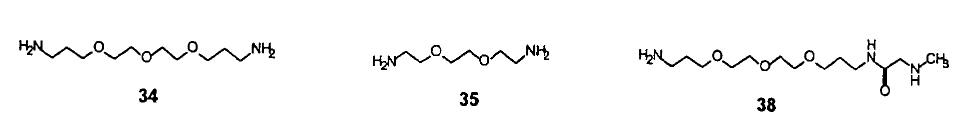

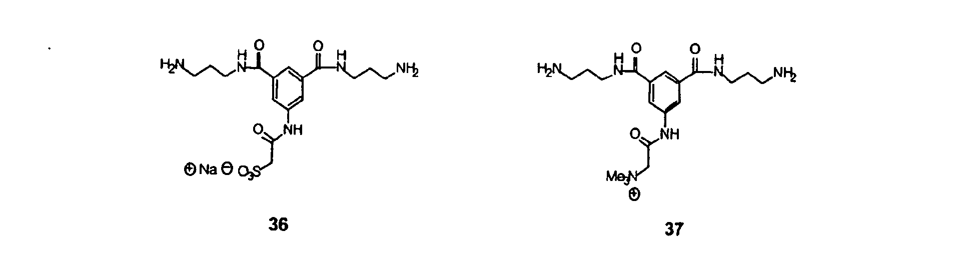

This invention discloses new chemical species that are composed of any combination of affinity ligands (e.g. biotin moieties), effector agents (e.g. radionuclide binding moieties), protein reactive moieties, trifunctional cross-linking moiety, and linking moieties. In specific examples, the reagents of this invention (generically shown in schematic structure (I)) provide a means of biotinylation and radiolabeling of biomolecules. This results in a minimally modified biomolecule (MMB). Irrespective of the individual components of the new chemical species, the process of conjugation and radiolabeling can occur by two distinctly different methods to give the same final product (the MMB), as depicted in Scheme(II) below. Path A is termed postformed conjugate(radio)labeling and Path B is termed preformed conjugate (radio)-labeling. Path A, where a compound of this invention is conjugated with the biomolecule first, and subsequently radiolabeled with the radionuclide chosen, is the preferred method of conjugation and radiolabeling. However, some radionuclide binding/bonding conditions are not compatible with certain biomolecules, therefore, Path B may be used as an alternative approach.

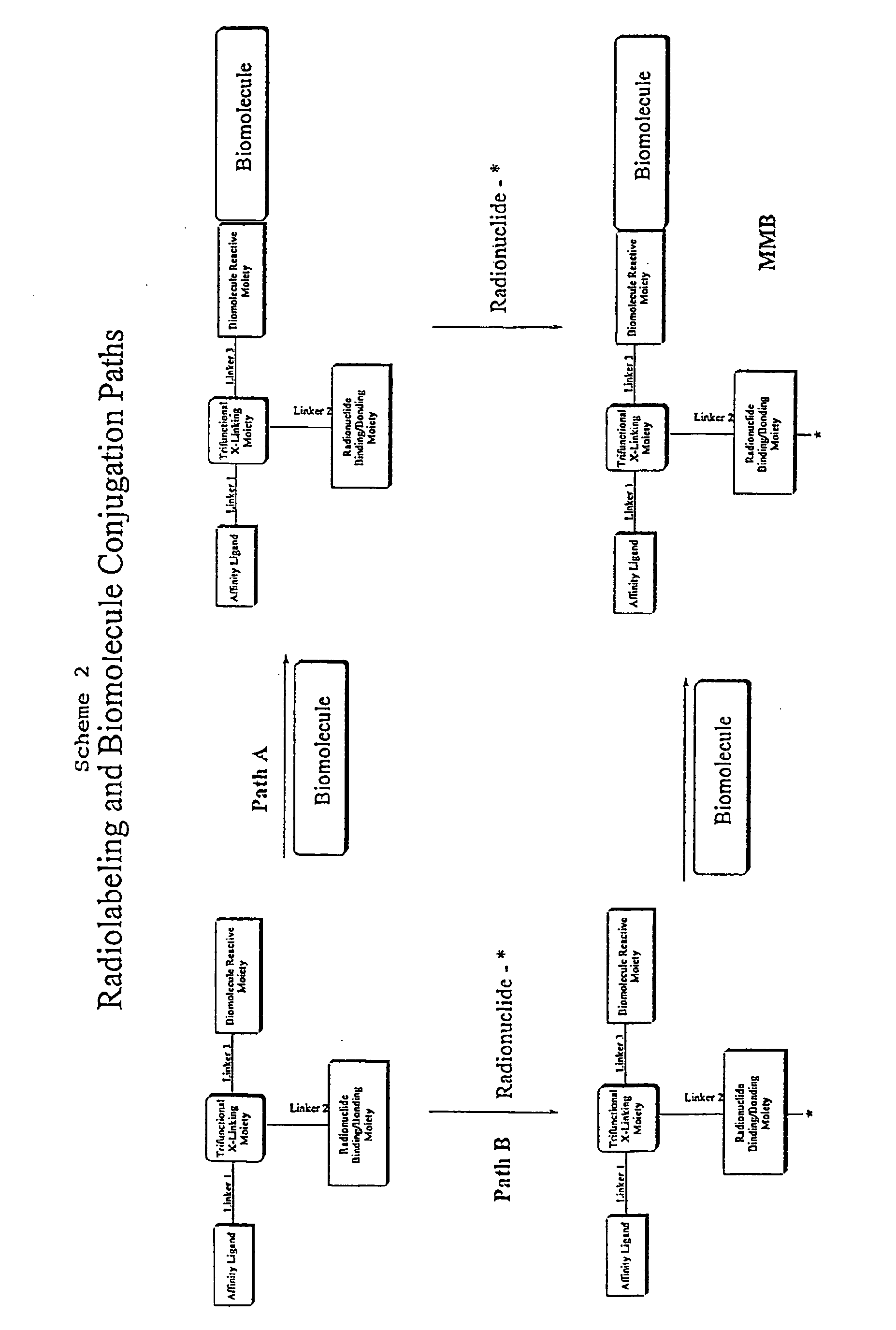

### EXAMPLES

The following examples 1-7 are provided to show some of the different combinations of reagents that are disclosed herein, and to show methods for preparing them. The examples are provided by way of illustration, not by way of limitation. Many further examples can be made by differing combinations of chemical moieties as depicted in the general formulation. The examples 1-6 are followed by reaction schemes relating to each example for the production of the reagents 39-44, wherein reagents 39 and 43 are reagents according to the present invention.

### Example 1

Compound 39 is a reagent according to the present invention and contains biotin as the biotin moiety; a biotinidase stabilized linker as linker 1; amino-isophthalic acid as the trifunctional cross-linking moiety; a CHX-DTPA group as a chelator for In-111 and Y-90; an aminobenzyl group for linker 2; no linker 3; and an isothiocyanate biomolecule reactive moiety. A method for synthesizing 39 from previously known reagents is provided.

### Example 2

Compound 40 is a reagent according to the present invention and contains biotin as the biotin moiety; a biotinidase stabilized (N-methyl) linker as linker 1; aminoisophthalic acid as the trifunctional cross-linking moiety; a tri-*n*-butylstannylbenzoate group as a moiety that is rapidly reacted to bond with the radiohalogens Br-75/76/77, I-123/124/125/131, or At-211; a trioxadiamine for linker 2; no linker 3; and a tetrafluorophenyl ester biomolecule reactive moiety. A method for synthesizing 40 from previously known reagents is provided.

### Example 3

Compound 41 is a reagent according to the present invention and contains homobiotin as the biotin moiety; a trioxadiamine linker as linker 1; aminoisophthalic acid as the trifunctional cross-linking moiety; an acid labile protected N₂S₂ group as a chelator for Tc-99m or Re-186/188; an propionate moiety for linker 2; no linker 3; and a tetrafluorophenyl ester biomolecule reactive moiety. A method for synthesizing 41 from previously known reagents is provided.

### Example 4

Compound 42 is a reagent according to the present invention and contains homobiotin as the biotin moiety; a trioxadiamine linker as linker 1; aminoisophthalic acid as the trifunctional cross-linking moiety; a BAT group as a chelator for Tc-99m or Re-186/188; a pentyloxybenzoate group for linker 2; no linker 3 and a tetrafluorophenyl ester biomolecule reactive moiety. This example is shown in that the BAT chelate allows the reagent to be coupled with a biomolecule (e.g. protein) prior to attaching the radionuclide. Modification Path A. A method for synthesizing 42 from previously known reagents is provided.

### Example 5

Compound 43 is a reagent according to the present invention and contains biotin as the biotin moiety; a biotinidase stabilized linker as linker 1; aminoisophthalic acid as the trifunctional cross-linking moiety; a TETA group as a chelator for Cu-67; an amibenzyl group for linker 2; no linker 3; and an isothiocyanate biomolecule reactive moiety. A method for synthesizing 43 from previously known reagents is provided.

### Example 6

Compound 44 is a reagent according to the present invention and contains homobiotin as the biotin moiety; a biotinidase stabilized linker as linker 1; tricarboxybenzene as the trifunctional cross-linking moiety; a tri-*n*-butylstannylbenzoate moiety for reaction with radiohalogens; a trioxadiamine moiety for linker 2; a trioxadiamine moiety for linker 3; and a maleimide group as the biomolecule reactive moiety. A method for synthesizing 44 from previously known reagents is provided.

### Example 7

Compound 45 is a reagent according to the present invention and contains biotin as the biotin moiety; a biotinidase stabilized linker (the glycyl moiety is replaced by an aspartyl moiety as linker 1; amino-isophthalic acid as the trifunctional cross-linking moiety; a CHX-A"-DTPA group as a chelator for In-111, Y-90 and Bi-213; an aminobenzyl group for linker 2; no linker 3; and an isothiocyanate biomolecule reactive moiety. The synthesis sequence of reactions to prepare this compound are shown in scheme 7.

### Example 1

### Reagent with Biotin, Biotinidase Stabilizing Linker, CHX-DTPA Chelate, and Isothiocyanate

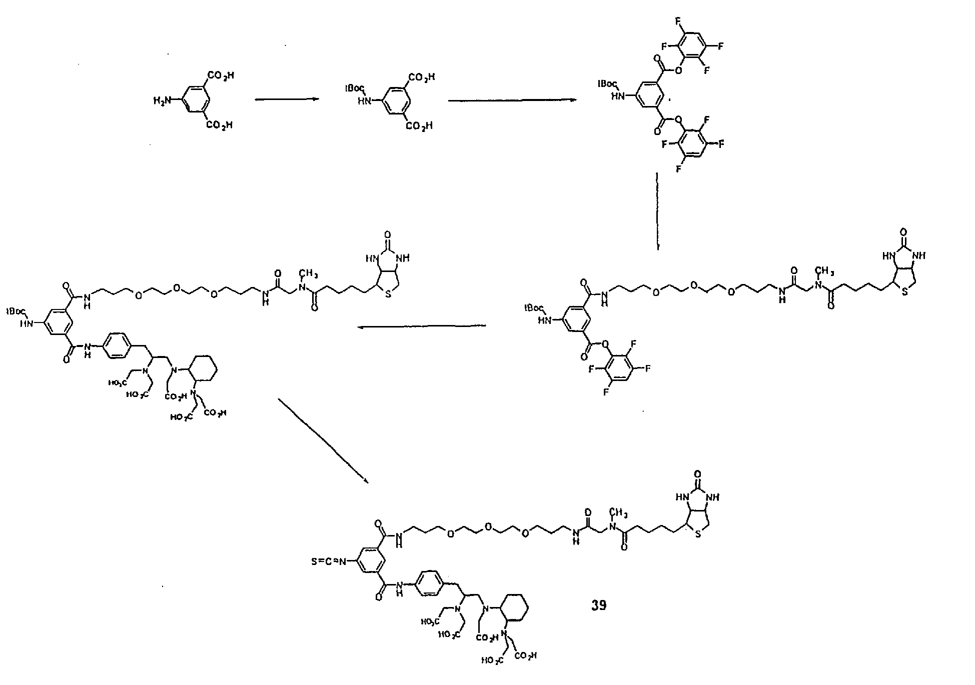

### Example 2

### Reagent with Biotin, Biotinidase Stabilized Linker, Arylstannane Radiohalogenation Moiety, and Tetrafluorophenyl Ester

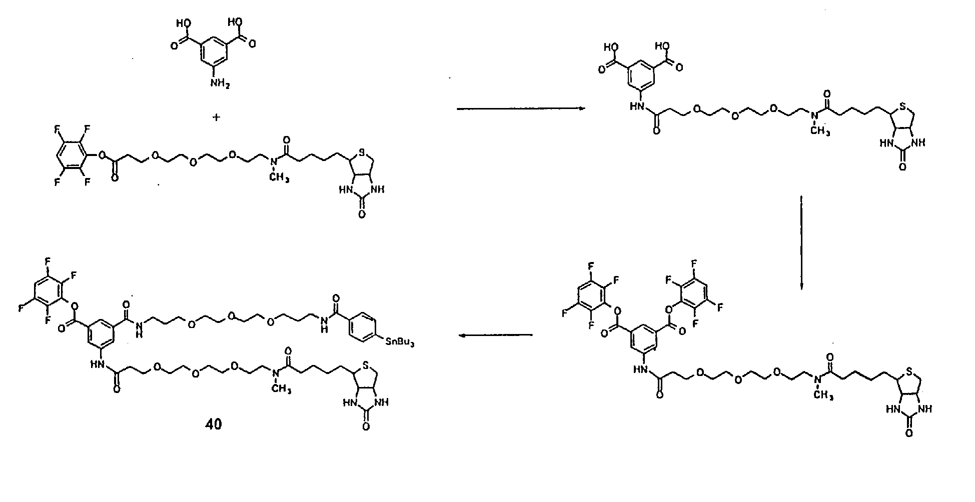

### Example 3

### Reagent with Homobiotin, DiamidoDithio (N ₂S₂) Chelate, and Tetrafluorophenyl Ester

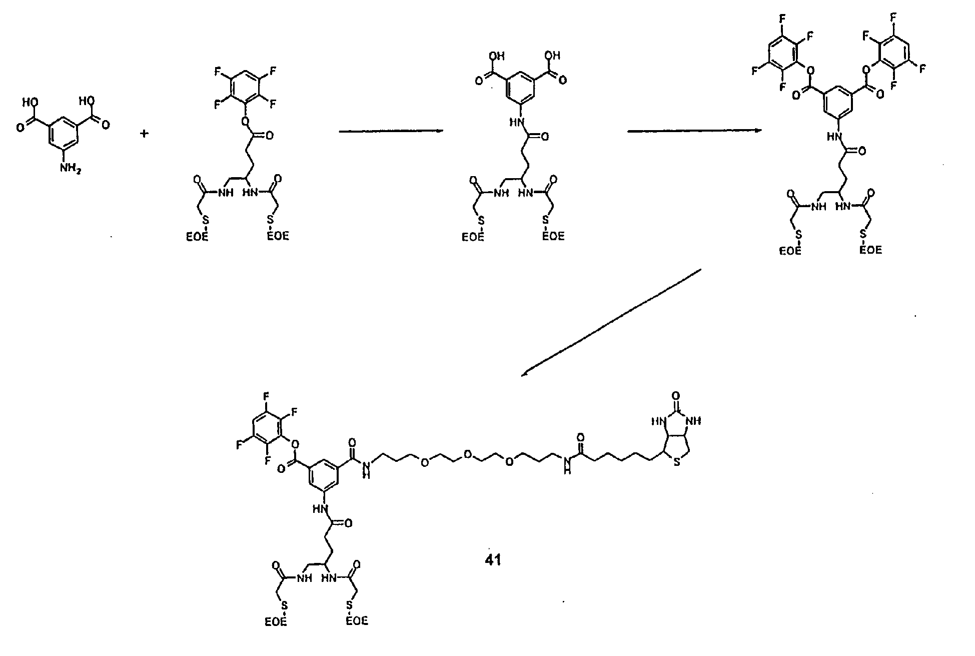

### Example 4

### Reagent with Homobiotin, DiaminoDithio (N ₂S₂) Chelate, and Tetrafluorophenyl Ester

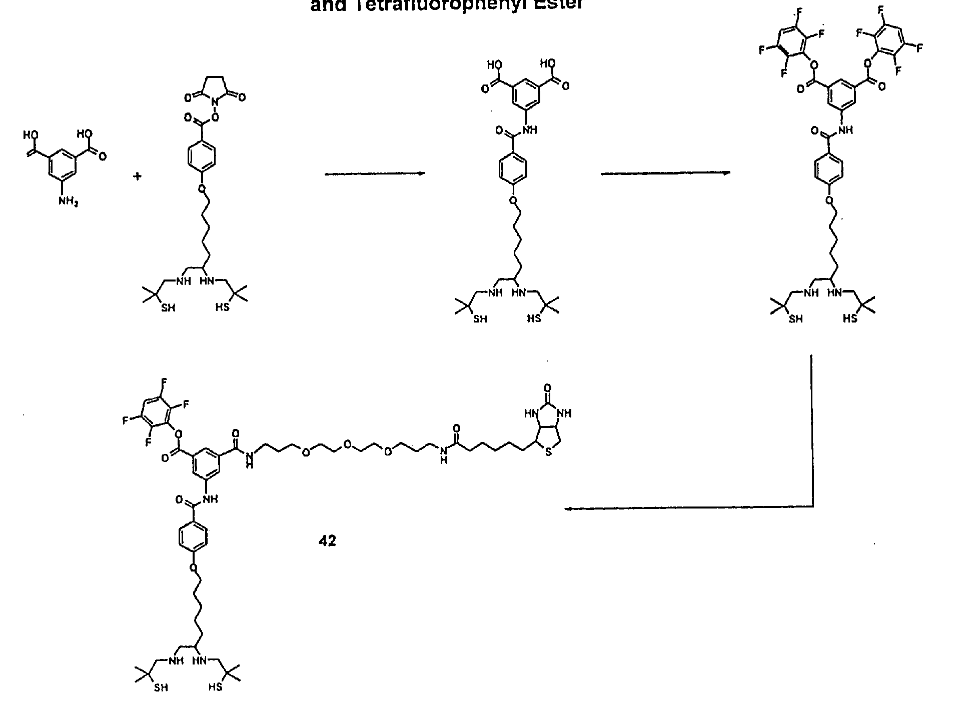

### Example 5

### Reagent with Biotin, Biotinidase Stabilizing Linker, TETA Chelate, and Isothiocyanate

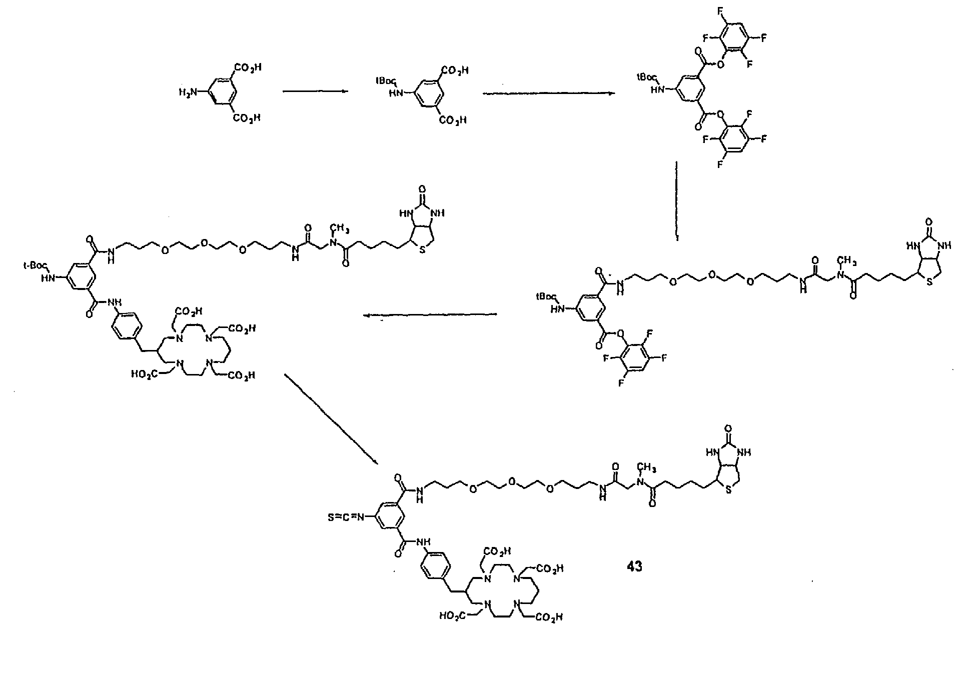

### Example 6

### Reagent with Homobiotin, Arylstannyl Radiohalogenation Moiety, and Maleimide

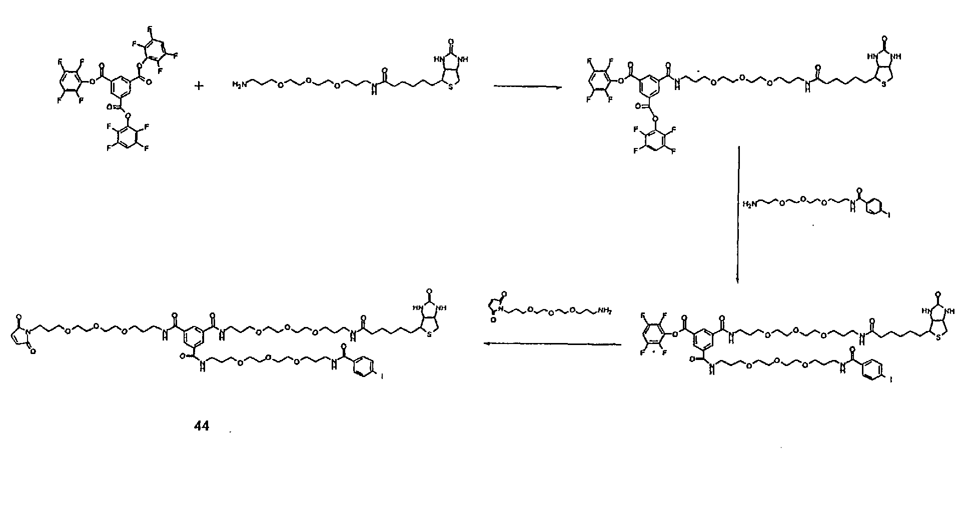

### Exempel 7

### Reagent with Biotin, Biotinidase Stabilizing Linker, CHX-A"-DTPA Chelate, and Isothiocyanate Conjugation Moiety

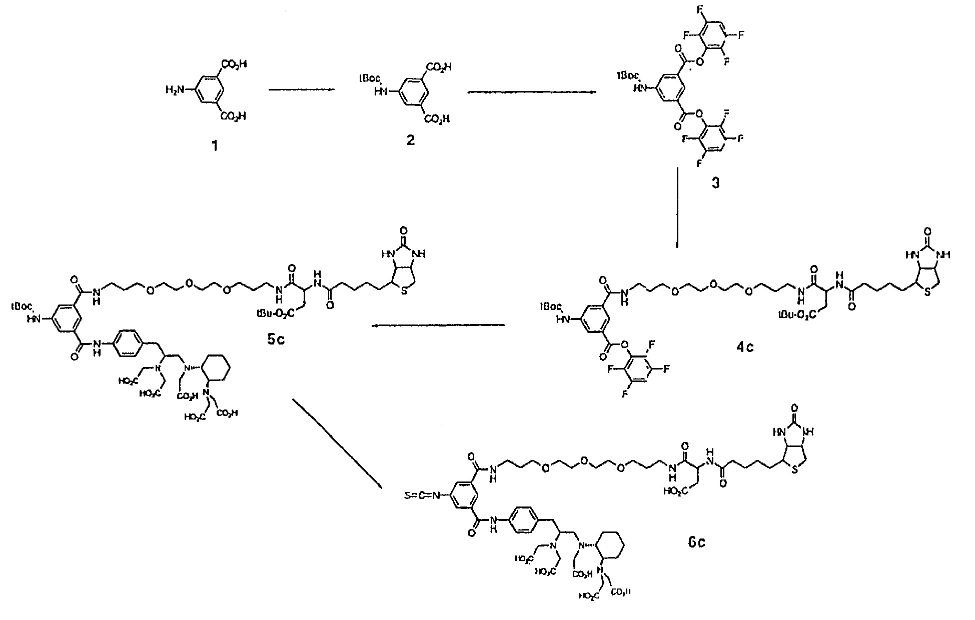

## Claims

1. A reagent for conjugation to a biomolecule, wherein the reagent is a single molecule with three different functional parts and has the following schematic structure (I): 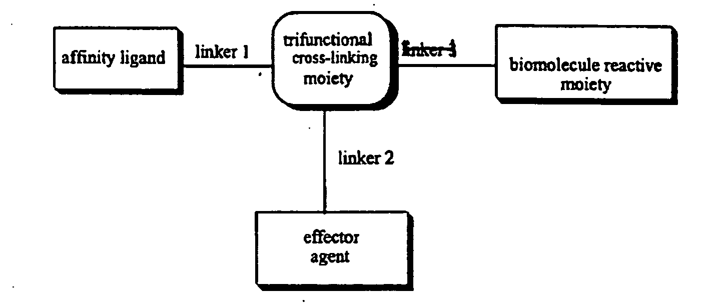
a) wherein a trifunctional cross-linking moiety chosen from the group consisting of triaminobenzene, tricarboxybenzene, dicarboxyaniline (aminoisophtaalic acid), and diaminobenzoic acid is coupled to
b) an affinity ligand via a linker 1, wherein said affinity ligand is chosen from the group consisting of biotin, norbiotin, homobiotin, oxybiotin, iminobiotin, desthiobiotin, diaminobiotin, biotinsulfoxide, and biotinsulfone, and wherein linker 1 contains ethers, thioethers, or ionisable groups such as carboxylates, sulfonates, and ammonium groups, and wherein when the affinity ligand is biotin, blockade of biotinidase activity has been provided by introducing an alpha carboxylate or N-methyl group in linker 1,
c) to an effector agent via a covalent bond, optionally via a linker 2, said effector agent exerting its effect on cells, tissues and/or humorous molecules in vivo, ex vivo or in vitro, and is chosen from the group consisting of radionuclide binding/bonding moieties comprising amino-carboxy derivatives or cyclic amines to which radionuclides have the ability to be bound or are bound by chelation, N₂S₂ and N₃S chelates for Tc and Re radionuclides, nonradioactive elements having the ability to be converted to radioactive elements, or photoactive compounds, wherein linker 2 contains ethers, thioethers, or ionisable groups such as carboxylates, sulfonates, and ammonium groups,
d) and to a biomolecule reactive moiety, said moiety is chosen from the group consisting of activated esters, aryl and alkyl imidates, alkyl or aryl isocyanates or isothiocyanates reacting with amino groups on the biomolecule, or maleimides or alpha-haloamides reacting with sulfhydryl groups on the biomolecule, or aryl or alkyl hydrazines, or alkyl or aryl hydroxylamines reacting with aldehyde or ketone groups naturally occuring or synthetically produced on the biomolecule.

2. The reagent according to claim 1, wherein the amino-carboxy derivatives are EDTA, DTPA, Me-DTPA, CITC-DTPA, and cyclohexyl-DTPA.

3. The reagent according to claim 1, wherein the cyclic amines are NOTA, DOTA, and TETA.

4. The reagent according to any one of the preceding claims, wherein the amino-carboxy derivatives and the cyclic amines in the effector agent are bound to an In, Y, Pb, Bi, Cu, Sm, or Lu radionuclide.

5. The reagent according to claim 1, wherein the effector agent is a compound having the ability to be converted to a photoactive compound.

6. The reagent according to claim 5, wherein the photoactive compound is a chromophore or a fluorophore.

7. The reagent according to claim 1, wherein the effector agent is provided with positron imaging radionuclides; therapeutic radionuclides; or gamma imaging radionuclides.

8. The reagent according to claim 7, wherein the positron imaging radionuclides are F-18, Br-75, Br-76 and I-24.

9. The reagent according to claim 8, wherein the therapeutic radionuclides are Y-90, I-131, In-114m, Re-186, Re-188, Cu-67, Sm-153, Lu-177, Bi-212, Bi-213, At-211, and Ra-223.

10. The reagent according to claim 7, wherein the gamma imaging radionuclides are Tc-99m, In-111, and I-123.

11. The reagent according to any one of the preceding claims, wherein linker 1 provides a spacer length of 6-20 atoms.

12. The reagent according to claim 11, wherein when the affinity ligand is biotin linker 1 has a length of 9 Å or more.

13. The reagent according to any one of the preceding claims, wherein linker 2 is excluded.

14. The reagent according to any one of claims 1-12, wherein linker 2 provides a spacer length of 1-25 atoms.

15. The reagent according to claim 14, wherein the spacer length is 6-18 atoms.

16. The reagent according to claim 1, wherein the activated esters are N-hydroxysuccinimide esters, sulfo-N-hydroxysuccinimide esters or phenolic esters.

17. The reagent according to any one of the previous claims, wherein it is chosen from the group consisting of the following compounds:

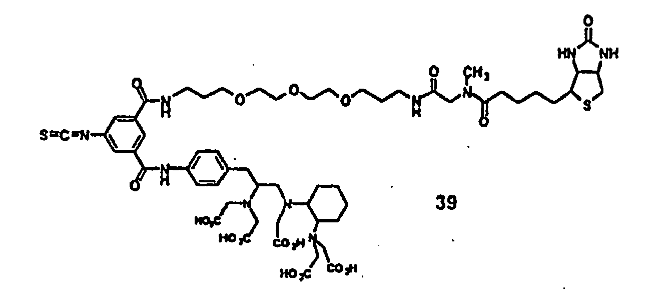

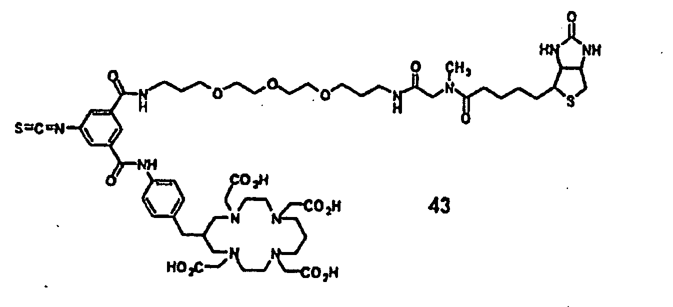

18. The reagent according to any one of the preceding claims for use as a pharmaceutical.

19. Use of the reagent according to any one of the preceding claims for the manufacture of a medicament for diagnosis and/or treatment of cancer.

20. Conjugate comprising the reagent according to any one of claims 1-18 bound to a biomolecule, wherein said biomolecule in the conjugate is minimally modified.

21. Conjugate according to claim 20, wherein the biomolecule is a protein, a peptide, an enzyme, a toxin, or a tumor binding monoclonal antibody.

22. Method for the production of a conjugate according to claims 20 and 21 comprising a minimally modified biomolecule with the desired biological properties retained, wherein a reagent according to any one of claims 1-18 is bound to a biomolecule as defined in any one of claims 20 and 21.

23. A method for the production of a radiolabelled conjugate, wherein a reagent according to any one of claims 1-18 provided with a radionuclide as defined in any one of claims 9 and 10 is conjugated to a biomolecule as defined in any one of claims 20 and 21, or alternatively, said reagent is conjugated to said biomolecule prior to attachment of said radionuclide.

24. Method for in vitro analysis of affinity labelled biomolecules, wherein the amount of affinity ligands on a conjugate comprising the reagent according to any one of claims 1-18 and a biomolecule is determined via the activity of a photoactive agent, wherein said amount is proportioned to the amount of the photoactive agent.

25. Kit for extracorporeally eliminating or at least reducing the concentration of a non-tissue-bound therapeutic or diagnostic biomolecule conjugate in the plasma or whole blood of a vertebrate host, said kit comprising
a) a therapeutic or diagnostic biomolecule,
b) a reagent according to any one of claims 1-18 for simultaneous conjugation of an affinity ligand and an effector agent to said biomolecule,
c) means for extracorporeal circulation of whole blood or plasma from a vertebrate host,
d) an optional plasma separation device for separation of plasma from blood,
e) an extracorporeal adsorption device, and
f) a means for return of whole blood or plasma without or with a low concentration of non-tissue-bound target specific therapeutic or diagnostic agent to the vertebrate host, wherein said adsorption device comprises immobilized receptors specific towards the affinity ligand.

## Patentansprüche

1. Reagenz für die Konjugation an ein Biomolekül,
wobei das Reagenz ein einzelnes Molekül mit drei verschiedenen funktionellen Teilen ist und folgende schematische Struktur (I) aufweist: 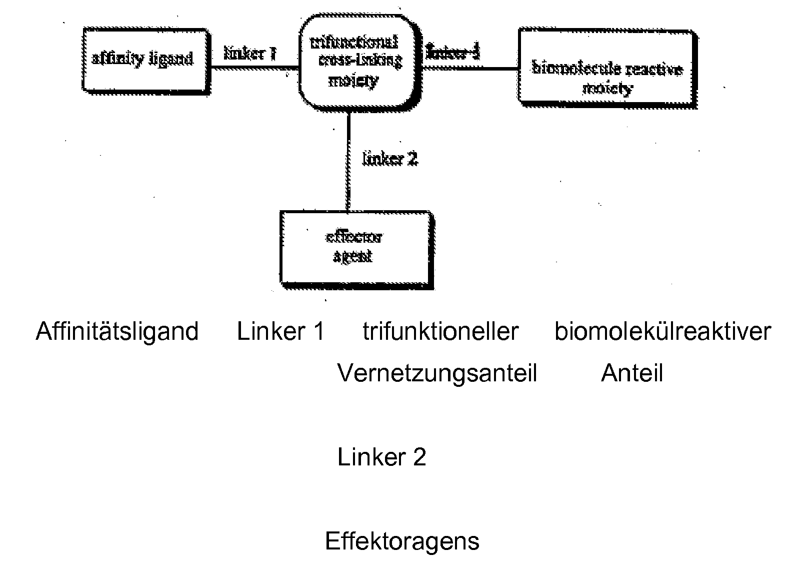
a) wobei ein trifunktifunktioneller Vernetzungsanteil ausgewählt aus der Gruppe bestehend aus Triaminobenzol, Tricarboxybenzol, Dicarboxyanilin(aminoisophthalsäure) und Diaminobenzoesäure an Folgendes gekoppelt wird
b) einen Affinitätsliganden über einen Linker 1, wobei der Affinitätsligand aus der Gruppe ausgewählt ist bestehend aus Biotin, Norbiotin, Homobiotin, Oxybiotin, Iminobiotin, Desthiobiotin, Diaminobiotin, Biotinsulfoxid und Biotinsulfon und wobei der Linker 1 Ether, Thiorether oder ionisierbare Gruppen wie Carboxylate, Sulfonate und Ammoniumgruppen enthält, und wobei, wenn der Affinitätsligand Biotin ist, die Blockade der Biotinidaseaktivität durch Einführen eines Alpha-Carboxylats oder einer N-Methylgruppe in den Linker 1 bereitgestellt worden ist,
c) an ein Effektoragens über eine kovalente Bindung, wahlweise über einen Linker 2, wobei das Effektoragens seine Wirkung auf Zellen, Gewebe und/oder humorale Moleküle in vivo, ex vivo oder in vitro ausübt und aus der Gruppe ausgewählt ist bestehend aus Radionuklidbindungs-/Verbindungsanteilen umfassend Aminocarboxyderivate oder cyclische Amine, an welche Radionuklide N₂S₂- und N₃S-Chelate für Tc- und Re-Radionuklide durch Chelieren gebunden werden können oder gebunden sind, wobei nichtradioaktive Elemente die Fähigkeit besitzen zu radioaktiven Elementen oder photoaktiven Verbindungen umgewandelt zu werden, wobei der Linker 2 Ether, Thioether oder ionisierbare Gruppen wie Carboxylate, Sulfonate und Ammoniumgruppen enthält,
d) und an einen biomolekülreaktiven Anteil, wobei der Anteil aus der Gruppe ausgewählt ist bestehend aus aktivierten Estern, Aryl- und Alkylimidaten, Alkyl- oder Arylisocyanaten oder - isothiocyanaten, die mit Aminogruppen am Biomolekül reagieren, oder Maleimiden oder Alpha-Haloamiden, die mit Sulfhydrylgruppen am Biomolekül reagieren, oder Aryl- oder Alkylhydrazinen oder Alkyl- oder Arylhydroxylaminen, die mit Aldehyd- oder Ketongruppen reagieren, die natürlich vorkommen oder am Biomolekül synthetisch erzeugt werden.

2. Reagenz nach Anspruch 1, wobei die Aminocarboxyderivate EDTA, DTPA, Me-DTPA, CITC-DTPA und Cyclohexyl-DTPA sind.

3. Reagenz nach Anspruch 1, wobei die cyclischen Amine NOTA, DOTA und TETA sind.

4. Reagenz nach einem der vorhergehenden Ansprüche , wobei die Aminocarboxyderivate und die cyclischen Amine in dem Efifektoragens an ein In-, Y-, Pb-, Bi-, Cu-, Sm- oder Lu-Radionuklid gebunden sind.

5. Reagenz nach Anspruch 1, wobei das Effektoragens eine Verbindung ist, die die Fähigkeit aufweist, in eine photoaktive Verbindung umgewandelt zu werden.

6. Reagenz nach Anspruch 5, wobei die photoaktive Verbindung ein Chromophor oder ein Fluorophor ist.

7. Reagenz nach Anspruch 1, wobei das Efifektoragens mit Positronbildgebungsradionukliden, therapeutischen Radionukliden oder Gamma-Bildgebungsnukliden ausgestattet ist.

8. Reagenz nach Anspruch 7, wobei die Positronbildgebungsradionuklide F-18, Br-76 und I-24 sind.

9. Reagenz nach Anspruch 8, wobei die therapeutischen Radionuklide Y-90, I-131, In-114m, Re-186, Re-188, Cu-67, Sm-153, Lu-177, Bi-212, Bi-213, At-211 und Ra-223 sind.

10. Reagenz nach Anspruch 7, wobei die Gamma-Bildgebungsnuklide Tc-99m, In-111 und I-123 sind.

11. Reagenz nach einem der vorhergehenden Ansprüche, wobei der Linker 1 eine Spacerlänge von 6-20 Atomen bereitstellt.

12. Reagenz nach Anspruch 11, wobei, wenn der Affinitätsligand Biotin ist, der Linker 1 eine Länge von 9 Å oder mehr aufweist.

13. Reagenz nach einem der vorhergehenden Ansprüche, wobei der Linker 2 ausgeschlossen ist.

14. Reagenz nach einem der vorhergehenden Ansprüche 1-12, wobei der Linker 2 eine Spacerlänge von 1-25 Atomen bereitstellt.

15. Reagenz nach Anspruch 14, wobei die Spacerlänge 6-18 Atome beträgt.

16. Reagenz nach Anspruch 1, wobei die aktivierten Ester N-Hydroxysuccinimidester, Sulfo-N-hydroxysuccinimidester oder Phenolester sind.

17. Reagenz nach einem der vorhergehenden Ansprüche, wobei es aus der Gruppe ausgewählt ist bestehend aus den folgenden Verbindungen:

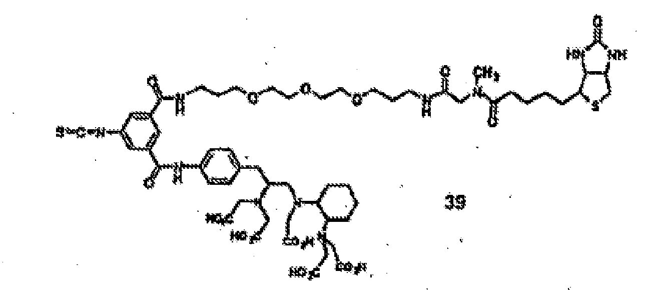

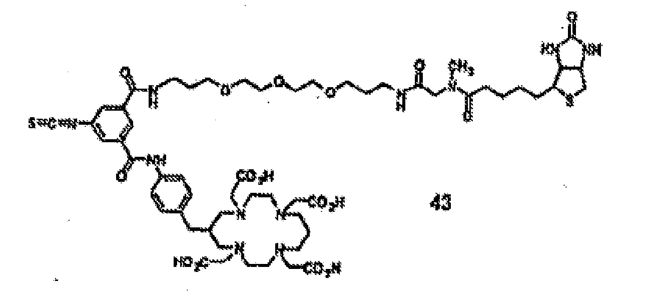

18. Reagenz nach einem der vorhergehenden Ansprüche zur Verwendung als Arzneimittel.

19. Verwendung des Reagenz nach einem der vorhergehenden Ansprüche für die Herstellung eines Medikaments für die Diagnose und/oder Behandlung von Krebs.

20. Konjugat umfassend das Reagenz nach einem der Ansprüche 1-18, das an ein Biomolekül gebunden ist, wobei das Biomolekül in dem Konjugat minimal modifiziert ist.

21. Konjugat nach Anspruch 20, wobei das Biomolekül ein Protein, ein Peptid, ein Enzym, ein Toxin oder ein tumorbindender monoklonaler Antikörper ist.

22. Verfahren für die Herstellung eines Konjugats nach den Ansprüchen 20 und 21, umfassend ein minimal modifiziertes Biomolekül, dessen erwünschte biologische Eigenschaften beibehalten worden sind, wobei ein Reagenz nach einem der Ansprüche 1-18 an ein Biomolekül wie in einem der Ansprüche 20 und 21 definiert gebunden ist.

23. Verfahren für die Herstellung eines radiomarkierten Konjugats, wobei ein Reagenz nach einem der Ansprüche 1-18, das mit einem Radionuklid wie in einem der Ansprüche 9 und 10 definiert versehen ist, an ein Biomolekül wie in irgendeinem der Ansprüche 20 und 21 definiert konjugiert wird oder alternativ das Reagenz an das Biomolekül vor dem Abknüpfen des Radionuklids konjugiert wird.

24. Verfahren für die In Vitro-Analyse von affinitätsmarkierten Biomolekülen , wobei die Menge von Affinitätsliganden an einem Konjugat, das das Reagenz nach einem der Ansprüche 1-18 und ein Biomolekül umfasst, durch die die Aktivität eines photoaktiven Mittels bestimmt wird, wobei die Menge der Menge des photoaktive Mittels proportional ist.

25. Kit zum extrakorporalen Eliminieren oder zumindest Reduzieren der Konzentration eines nicht gewebegebundenen therapeutischen oder diagnostischen Biomolekülkonjugats im Plasma oder Vollblut eines Vertebratenwirts, wobei der Kit Folgendes umfasst
a) ein therapeutisches oder diagnostisches Biomolekül,
b) ein Reagenz nach einem der Ansprüche 1-18 für das gleichzeitige Konjugieren eines Affinitätsliganden und eines Effektoragens an das Biomolekül,
c) eine Möglichkeit für das extrakorporale Zirkulieren von Vollblut oder Plasma von einem Vertebratenwirt,
d) ein wahlweises Plasmatrennungsgerät zum Trennen von Plasma von Blut,
e) ein extrakorporales Adsorptionsgerät und
f) eine Möglichkeit für das Rückführen von Vollblut oder Plasma
ohne oder mit einer niedrigen Konzentration an nicht gewebegebundenem, target-spezifischem therapeutischem oder diagnostischem Mittel zum Vertebratenwirt, wobei das Adsorptionsgerät immobilisierte Rezeptoren umfasst, die dem Affinitätsliganden gegenüber spezifisch sind.

## Revendications

1. Réactif pour la conjugaison avec une biomolécule,
le réactif étant une molécule unique avec trois parties fonctionnelles différentes et ayant la structure schématique (I) suivante : 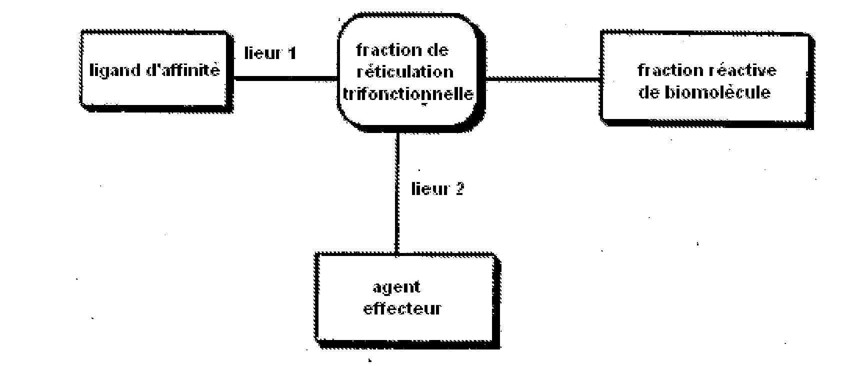
a) dans laquelle une fraction de réticulation trifonctionnelle choisie dans le groupe constitué par le triaminobenzène, le tricarboxybenzène, la dicarboxyaniline (acide aminoisophtalique), et un acide diaminobenzoïque est couplée à
b) un ligand d'affinité via un lieur 1, ledit ligand d'affinité étant choisi dans le groupe constitué par la biotine, la norbiotine, l'homobiotine, l'oxybiotine, l'iminobiotine, la desthiobiotine, la diaminobiotine, la biotine sulfoxyde, et la biotine sulfone, et le lieur 1 contenant des éthers, des thioéthers, ou des groupes ionisables tels que des carboxylates, des sulfonates, et des groupes ammonium, et dans lequel lorsque le ligand d'affinité est la biotine, le blocage de l'activité biotinidase a été permis par l'introduction d'un groupe alpha carboxylate ou N-méthyle dans le lieur 1,
c) à un agent effecteur via une liaison covalente, facultativement via un lieur 2, ledit agent effecteur exerçant son effet sur les cellules, les tissus et/ou les molécules humorales in vivo, ex vivo ou in vitro, et étant choisi dans le groupe constitué par des fractions de liaison aux radionucléides comprenant des dérivés amino-carboxy ou des amines cycliques auxquels les radionucléides ont la capacité de se lier ou sont liés par chélation, des chélates N₂S₂ et N₃S pour les radionucléides Tc et Re, des éléments non radioactifs ayant la capacité de se convertir en éléments radioactifs, ou des composés photoactifs, le lieur 2 contenant des éthers, des thioéthers, ou des groupes ionisables tels que des carboxylates, des sulfonates, et des groupes ammonium,
d) et à une fraction réactive de biomolécule, ladite fraction étant choisie dans le groupe constitué par les esters actifs, les aryl et alkyl imidates, les alkyl ou aryl isocyanates ou isothiocyanates réagissant avec les groupes amino présents sur la biomolécule, ou des maléimides ou des alpha-haloamides réagissant avec les groupes sulfhydryle sur la biomolécule, ou des aryl ou alkyl hydrazines, ou des alkyl ou aryl hydroxylamines réagissant avec des groupes aldéhyde ou cétone naturels ou synthétiques sur la biomolécule.

2. Réactif selon la revendication 1, dans lequel les dérivés amino-carboxy sont l'EDTA, le DTPA, le Me-DTPA, le CITC-DTPA, et le cyclohexyl-DTPA.

3. Réactif selon la revendication 1, dans lequel les amines cycliques sont les NOTA, DOTA, et TETA.

4. Réactif selon l'une quelconque des revendications précédentes, dans lequel les dérivés amino-carboxy et les amines cycliques dans l'agent effecteur sont liés à un radionucléide In, Y, Pb, Bi, Cu, Sm, ou Lu.

5. Réactif selon la revendication 1, dans lequel l'agent effecteur est un composé ayant la capacité d'être converti en un composé photoactif.

6. Réactif selon la revendication 5, dans lequel le composé photoactif est un chromophore ou un fluorophore.

7. Réactif selon la revendication 1, dans lequel l'agent effecteur est doté de radionucléides pour l'imagerie par émission de positrons ; de radionucléides thérapeutiques ; ou de radionucléides pour l'imagerie gamma.

8. Réactif selon la revendication 7, dans lequel les radionucléides pour l'imagerie par émission de positrons sont les F-18, Br-75, Br-76 et I-24.

9. Réactif selon la revendication 8, dans lequel les radionucléides thérapeutiques sont les Y-90, I-131, In-114m, Re-186, Re-188, Cu-67, Sm-153, Lu-177, Bi-212, Bi-213, At-211, et Ra-223.

10. Réactif selon la revendication 7, dans lequel les radionucléides pour l'imagerie gamma sont les Tc-99m, In-111 et I-123.

11. Réactif selon l'une quelconque des revendications précédentes, dans lequel le lieur 1 fournit une longueur d'espaceur de 6 à 20 atomes.

12. Réactif selon la revendication 11, dans lequel lorsque le ligand d'affinité est la biotine, le lieur 1 a une longueur de 9 Å ou moins.

13. Réactif selon l'une quelconque des revendications précédentes, dans lequel le lieur 2 est exclu.

14. Réactif selon l'une quelconque des revendications 1 à 12, dans lequel le lieur 2 fournit une longueur d'espaceur de 1 à 25 atomes.

15. Réactif selon la revendication 14, dans lequel la longueur d'espaceur est de 6 à 18 atomes.

16. Réactif selon la revendication 1, dans lequel les esters actifs sont des esters de N-hydroxysuccinimide, des esters de sulfo-N-hydroxysuccinimide ou des esters phénoliques.

17. Réactif selon l'une quelconque des revendications précédentes, dans lequel ledit réactif est choisi dans le groupe constitué par les composés suivants :

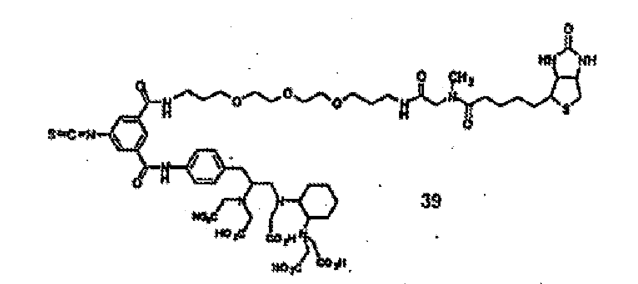

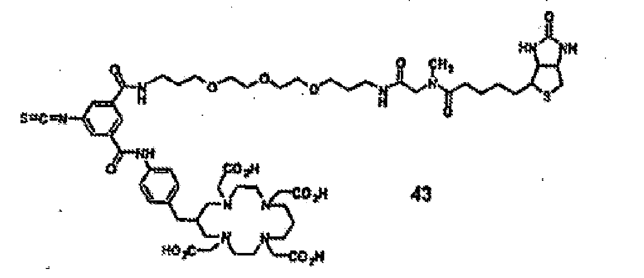

18. Réactif selon l'une quelconque des revendications précédentes, destiné à être utilisé en tant qu'un produit pharmaceutique.

19. Utilisation du réactif selon l'une quelconque des revendications précédentes, pour la fabrication d'un médicament destiné au diagnostic et/ou au traitement d'un cancer.

20. Conjugué comprenant le réactif selon l'une quelconque des revendications 1 à 18 lié à une biomolécule, ladite biomolécule dans le conjugué portant une modification minimale.

21. Conjugué selon la revendication 20, dans lequel la biomolécule est une protéine, un peptide, une enzyme, une toxine, ou un anticorps monoclonal se liant aux tumeurs.

22. Procédé de production d'un conjugué selon les revendications 20 et 21, qui comprend une biomolécule portant une modification minimale et conservant les propriétés biologiques souhaitées, un réactif selon l'une quelconque des revendications 1 à 18 étant lié à une biomolécule telle que définie dans l'une quelconque des revendications 20 et 21.

23. Procédé de production d'un conjugué radiomarqué, dans lequel un réactif selon l'une quelconque des revendications 1 à 18 doté d'un radionucléide tel que défini dans l'une quelconque des revendications 9 et 10 est conjugué à une biomolécule telle que définie dans l'une quelconque des revendications 20 et 21, ou en variante, ledit réactif est conjugué à ladite biomolécule avant la fixation dudit radionucléide.

24. Procédé d'analyse in vitro de l'affinité de biomolécules marquées, dans lequel la quantité de ligands d'affinité sur un conjugué comprenant le réactif selon l'une quelconque des revendications 1 à 18 et une biomolécule est déterminée via l'activité d'un agent photoactif, ladite quantité étant proportionnelle à la quantité de l'agent photoactif.

25. Kit d'élimination extracorporelle ou au moins de réduction de la concentration d'un conjugué de biomolécule diagnostique ou thérapeutique non lié au tissu dans le plasma ou le sang total d'un hôte vertébré, ledit kit comprenant
a) une biomolécule thérapeutique ou diagnostique,
b) un réactif selon l'une quelconque des revendications 1 à 18 pour la conjugaison simultanée d'un ligand d'affinité et d'un agent effecteur à ladite biomolécule,
c) des moyens pour la circulation extracorporelle du sang total ou du plasma d'un hôte vertébré,
d) un dispositif de séparation du plasma facultatif pour séparer le plasma du sang,
e) un dispositif d'adsorption extracorporel, et
f) des moyens pour renvoyer dans le corps de l'hôte vertébré le sang total ou le plasma
sans ou avec une concentration faible de l'agent diagnostique ou thérapeutique spécifique cible non lié aux tissus, ledit dispositif d'adsorption comprenant des récepteurs immobilisés spécifiques du ligand d'affinité.
